(19) Europäisches Patentamt
European Patent Office
Office européen des brevets

(11) Publication number: **0 119 449 A1**

# (12) EUROPEAN PATENT APPLICATION

(21) Application number: 84101394.9

(22) Date of filing: 10.02.84

(51) Int. Cl.³: C 07 D 231/06, A 61 K 31/415

(30) Priority: 11.02.83 GB 8303782

(43) Date of publication of application: 26.09.84 Bulletin 84/39

(84) Designated Contracting States: AT BE CH DE FR GB IT LI LU NL SE

(71) Applicant: THE WELLCOME FOUNDATION LIMITED, 183-193 Euston Road, London NW1 2BP (GB)

(72) Inventor: Islip, Peter John, 35 Arkwright Road, Sanderstead Surrey (GB)
Inventor: Copp, Frederick Charles, "Rotherwood" 32 Stanley Avenue, Beckenham Kent (GB)
Inventor: Kneen, Geoffrey, 119 Lennard Road, Beckenham Kent (GB)

(74) Representative: Sandmair, Kurt, Dr. et al, Dr. Berg, Dipl.-Ing. Stapf Dipl.-Ing. Schwabe, Dr. Dr. Sandmair Mauerkircherstrasse 45, D-8000 München 80 (DE)

## (54) Aminopyrazoline derivatives.

(57) The compounds of formula (I) and their pharmacologically acceptable acid addition salts are useful in medicine. In formula (I)

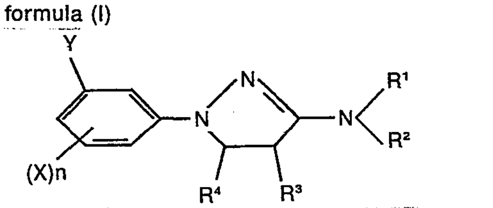

(I)

Y is a group selected from alkylthio, alkenylthio, arylthio, alkylsulphinyl, alkenylsulphinyl, arylsulphinyl, alkylsulphonyl, alkenylsulphonyl, arylsulphonyl, alkoxy, alkenyloxy, or aryloxy, in which group any hydrogen atom is optionally replaced by a halogen atom;

each X is the same or different and is independently selected from alkylthio, alkenylthio, arylthio, alkylsulphinyl, arylsulphinyl, alkenylsulphinyl, alkylsulphonyl, alkenylsulphonyl, arylsulphonyl, alkoxy, alkenyloxy, aryloxy, alkyl, alkenyl, aryl, trihaloalkyl, or halogen;

n is 0 to 4;

$R^1$ is hydrogen, alkyl, alkenyl, alkynyl, aralkyl or aryl;

$R^2$ is hydrogen, alkyl, acyl of 1–4 carbon atoms, aroyl of 6–10 carbon atoms or alkoxycarbonyl; or

$R^1$ and $R^2$ together represent

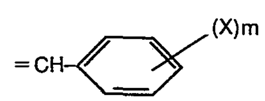

where X is a suitable substituent and m is 0–5; and

$R^3$ and $R^4$ are the same or different and each represents hydrogen or alkyl

provided that when $R^1$ is other than hydrogen, $R^2$ is not acyl or aroyl.

EP 0 119 449 A1

TITLE MODIFIED
see front page

## HETEROCYCLIC COMPOUNDS

This invention relates to certain novel substituted pyrazoline compounds, their preparation, formulations containing them, and the use of such compounds or formulations in medicine.

1-Phenyl-2-pyrazolines wherein the phenyl ring is substituted in the 3-position by a t-butyl, carboxy or trifluoromethyl group, or in the 4-position by a halo or methoxy group, are disclosed as anti-inflammatory agents in European Patent Specification 0 055 418.

We have now found that the novel 1-phenyl-2-pyrazolines of formula (I) (as defined hereinbelow) having one of a certain class of sulphur or oxygen-containing substituents in the 3-position of the phenyl ring and, optionally one or more further substituents unexpectedly inhibit the lipoxygenase and cyclooxygenase pathways of arachidonic acid metabolism both _in vitro_ and _in vivo_. These novel compounds are useful as anti-inflammatory, anti-allergic, analgesic or anti-pyretic agents, or agents for use in the prevention of tissue rejection.

Accordingly, the present invention provides a compound of formula (I)

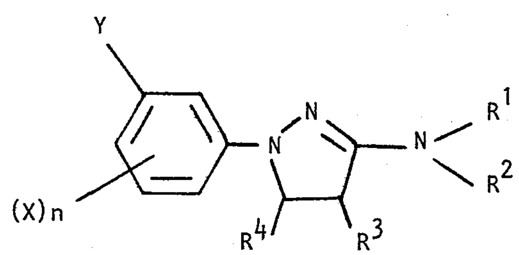

(I)

wherein

Y is a group selected from alkylthio, alkenylthio, arylthio, alkylsulphinyl, alkenylsulphinyl, arylsulphinyl, alkylsulphonyl, alkenylsulphonyl, arylsulphonyl, alkoxy, alkenyloxy, or aryloxy, in which group any hydrogen atom is optionally replaced by a halogen atom;

each X is the same or different and is independently selected from alkylthio, alkenylthio, arylthio, alkylsulphinyl, arylsulphinyl, alkenylsulphinyl, alkylsulphonyl, alkenylsulphonyl, arylsulphonyl, alkoxy, alkenyloxy, aryloxy, alkyl, alkenyl, aryl, trihaloalkyl, or halogen;

n is 0 to 4;

$R^1$ is hydrogen, alkyl, alkenyl, alkynyl, aralkyl or aryl;

$R^2$ is hydrogen, alkyl, acyl of 1-4 carbon atoms, aroyl of 6-10 carbon atoms or alkoxycarbonyl; or

$R^1$ and $R^2$ together represent =CH- 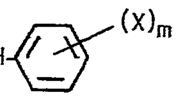 where X is a suitable substituent and m is 0-5; and

$R^3$ and $R^4$ are the same or different and each represents hydrogen or alkyl

provided that when $R^1$ is other than hydrogen, $R^2$ is not acyl or aroyl;

and the pharmacologically acceptable acid addition salts thereof.

In formula (I) and throughout the specification the following definitions apply unless the context otherwise requires:-

(a) The term "alkyl", either alone, or when included as part of another group, for example, alkoxy, shall be taken to refer to an alkyl or cycloalkyl group having from 1 to 6 carbon atoms, and, preferably, 1 to 4 carbon atoms.

(b) The terms alkenyl and alkynyl either alone or as part of another group shall be taken to refer to alkenyl and alkynyl groups each of which has from 3 to 6 carbon atoms, and preferably 3 or 4 carbon atoms.

(c) The term "acyl" shall be taken to refer to a radical of formula $R^aCO$-wherein $R^a$ is hydrogen or an aliphatic moiety. Examples of acyl groups include formyl, acetyl and propionyl.

(d) The term "aroyl" shall be taken to refer to a radical of formula $R^bCO$-wherein $R^b$ is an aryl or aryl-containing moiety such as phenyl or benzyl.

(e) The term "aralkyl" shall be taken to refer to an alkyl group substituted by one or more aryl groups.

(f) The term "aryl group", either alone or as part of another group, for example aralkyl, shall unless otherwise specified be taken to refer to an monocyclic or polycyclic aromatic or heteroaromatic ring system of from 5 to 10 atoms, selected from carbon, oxygen, sulphur and nitrogen, which may optionally be substituted by one or more suitable substituents. Examples of aryl groups include, phenyl, pyridyl, quinolyl and naphthyl; particularly preferred is phenyl.

(g) "Suitable substituents" include those known to persons skilled in the art for the substitution of aromatic ring systems, for example, halo, alkyl, trihaloalkyl, carboxy, alkoxy, hydroxy, alkylthio, amino (optionally substituted by one or more alkyl groups), alkysulphinyl and alkylsulphonyl (the alkyl group of which may optionally be substituted by one or more halogen atoms).

Since the compounds of formula (I) inhibit the cyclooxygenase pathway of arachidonic acid metabolism, they exhibit some of the properties of the non-steroidal (or aspirin-like) anti-inflammatory drugs, such as reduction of erythema, oedema and pain associated with prostaglandins leading to good symptomatic relief. However, aspirin-like drugs are not effective against the leucocyte-mediated aspects of chronic inflammatory disease, since they do not inhibit leukcotriene formation.

Anti-inflammatory corticosteroids indirectly inhibit the production of both prostaglandins and leukotrienes which could, in part, account for their superior therapeutic effects. However, steroid treatment is associated with serious local or systemic side-effects and prolonged administration is contra-indicated in many inflammatory conditions.

The present invention fulfils the need for a non-steroid anti-inflammatory compound which inhibits the synthesis of leukotrienes and prostaglandins, and which is free from steroid-related toxicity.

In addition, the compounds of formula (I) exhibit low levels of toxicity and favourable therapeutic indices. Compounds of formula (I), or pharmaceutically acceptable formulations thereof, may therefore be administered at low dosages to achieve a therapeutically effective level of anti-inflammatory activity.

Included within the scope of the compounds of formula (I) and their pharmacologically acceptable acid addition salts are those wherein $R^1$ is other than aralkyl and $R^2$ is other than aroyl. Within this subclass are those compounds and salts wherein both $R^1$ and $R^2$ are hydrogen, and also those where Y is alkylthio and/or n is 0, and $R^3$ and $R^4$ are preferably hydrogen.

Preferred compounds of formula (I) and their pharmacologically acceptable acid addition salts include those wherein Y is alkylthio or trihalo-alkysulphonyl, n is 0, $R^1$ is other than aryl, $R^2$ is other than alkoxycarbonyl and $R^3$ and $R^4$ are both hydrogen.

Particularly preferred are those compounds and salts wherein Y is alkylthio, n is 0, $R^1$ is hydrogen, alkyl, alkenyl, alkynyl, or benzyl (wherein benzyl is optionally substituted in the benzene ring by one or more suitable substituents such as alkoxy, eg. methoxy) $R^2$ is other than alkoxycarbonyl and $R^3$ and $R^4$ are both hydrogen.

An especially preferred compound of formula (I) is that compound where n is 0, Y is methylthio, and $R^1$, $R^2$, $R^3$ and $R^4$ are each hydrogen ie. 1-(3-methylthiophenyl)-3-amino-2-pyrazoline, and its pharmacologically acceptable acid addtion salts.

Other compounds of formula (I) include the following compounds and their pharmacologically acceptable acid addition salts:-

N-[1-(3-methylthiophenyl)-2-pyrazolin-3-yl]formamide
3-methylamino-1-(3-methylthiophenyl)-2-pyrazoline
3-amino-1-[3-(trifluoromethylsulphonyl)phenyl]-2-pyrazoline
3-amino-1-(3-methoxyphenyl)-2-pyrazoline
N-[1-(3-methylthiophenyl)-2-pyrazolin-3-y] acetamide
1-(3-methylthiophenyl)-3-(2-propynylamino)-2-pyrazoline
1-(3-methylthiophenyl)-3-(2-propenylamino)-2-pyrazoline
3-(4-methoxybenzylamino)-1-(3-methylthiophenyl)-2-pyrazoline

When used in medicine, the acid addition salts of a compound of formula (I) should be both pharmacologically and pharmaceutically acceptable acid addition salts, but non-acceptable salts may conveniently be used to prepare the bases of such acceptable salts and are not excluded from the scope of this invention. Acceptable salts may be derived from organic acids, particularly dicarboxylic acids. Such pharmacologically and pharmaceutically acceptable salts include those prepared

from the following acids: hydrochloric, hydrobromic, sulphuric, nitric, phosphoric, oxalic, fumaric, maleic, glycolic, salicylic, succinic, toluene-p-sulphonic, tartaric, acetic, citric, methane sulphonic, formic, benzoic, malonic, naphthalene-2-sulphonic and benzene sulphonic.

The compounds of formula (I) may be prepared by any method analogous to those known in the art, for example by the method of G.F.Duffin and J.D.Kendall in J.Chem.Soc. (1954), 408-415. Other methods include:

(1) Cyclisation and elimination of water from a compound of formula (II)

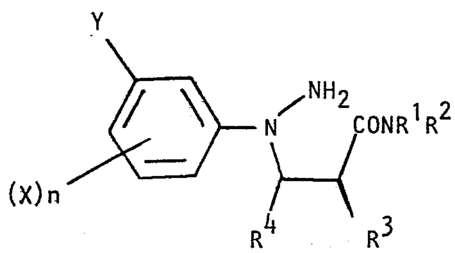

(II)

wherein $R^1$, $R^2$, $R^3$, $R^4$, Y, X and n as defined in formula (I). Suitable agents for the above cyclisation and elimination include phosphorus oxychloride ($POCl_3$).

The compound of formula (II) may itself be prepared by reaction of the corresponding compound of formula (III) with the corresponding compound of formula (IV).

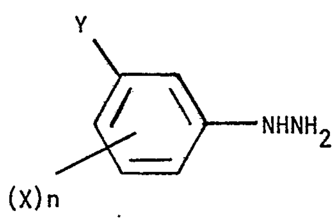

(III)

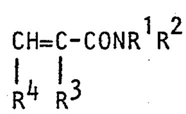

(IV)

where Y, X, n, $R^1$, $R^2$, $R^3$ and $R^4$ are as defined in formula (II).

This process is particularly applicable to the preparation of compounds of formula (I) where either $R^1$ is other than hydrogen or, when $R^1$ is hydrogen, it is sterically hindered by $R^2$ which is therefore a group such as tert-butyl.

(2) Another method for the preparation of compounds of formula (I) wherein $R^1$ is hydrogen comprises reaction of a compound of formula (VI) with a compound of formula (V):

$R^2$–Z (V)

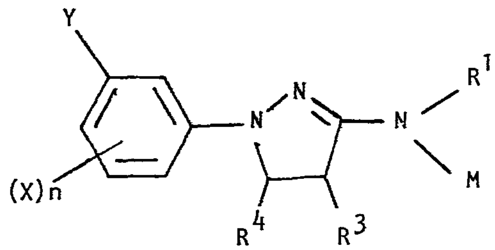

(VI)

wherein X, Y, n, $R^3$, $R^2$ and $R^4$ are as defined in formula (I)and $R^1$ is H; Z is a standard leaving group e.g. halo e.g. chloro or bromo; and M is an alkali or alkaline earth metal.

The reaction is preferably carried out at from 0 to 30°, more preferably at from 0 to 10°C. The compound of formula (VI) is preferably prepared by metallating the corresponding compound of formula (VIA):

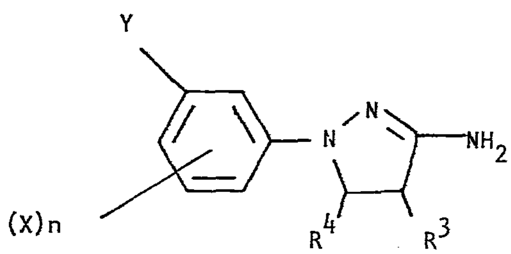

(VIA)

wherein Y, X, n, $R^3$ and $R^4$ are as defined in formula (VI). Suitable metallating agents include an alkyl alkali metal such as butyl lithium and the reaction preferably carried out at from -70°C to room temperature, more preferably at about -40°C. The compound of formula (VI) need not be isolated but may be converted to the compound of formula (I) in situ.

(3) A further method, wherein, in formula (I), $R^1$ is H and $R^2$ is other than a group having a terminal acetylenic hydrogen atom, comprises reaction of a compound of formula (VII) with $R^8$ M:

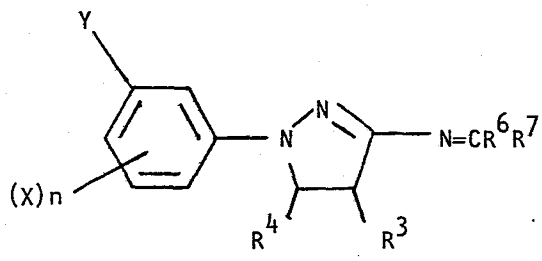

(VII)

wherein Y, X, n, $R^3$ and $R^4$ are as defined in formula (I); M is an alkali metal or an alkaline earth metal halide; and $-CR^6R^7R^8$ is $R^2$ as defined in formula (I). For example, $R^8M$ is an alkyl alkaline earth metal halide such as a Grignard reagent for example methylmagnesium iodide; or an alkyl alkali metal such as butyl lithium. For example, a method of preparing a compound of formula (I) wherein $R^2$ is isopropyl comprises reaction of a compound of formula (VII) wherein $R^6$ is H and $R^7$ is methyl with methyl magnesium iodide.

(4) A further method wherein $R^1$ is hydrogen comprises reduction of a compound of formula (VII) wherein Y, X, n, $R^3$ and $R^4$ are as defined in formula (I); and $-CHR^6R^7$ is $R^2$ as defined in formula (I). Suitable reducing agents are known to those skilled in the art and include sodium borohydride or another metallic reducing agent such as sodium cyano-borohydride; or, where $R^2$ does not include a -C=C- or a -C≡C- group by catalytic reduction or, where X includes halo, by non-catalytic agents such as sodium borohydride.

(5) A further method, wherein, in formula (I), $R^1$ is hydrogen and $R^2$ includes a methylene adjacent the nitrogen atom and preferably does not include alkenyl or alkynyl, comprises reduction of a compound of formula (VIII).

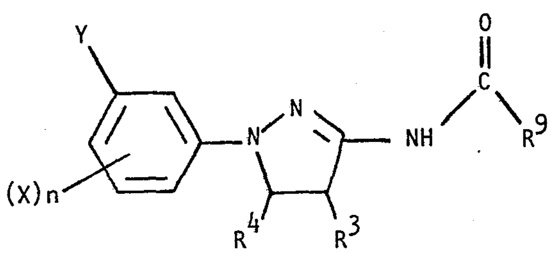

(VIII)

wherein X, Y, n, $R^3$, $R^4$ are as defined in formula (I) and either $CH_2R^9$ is $R^2$ as defined in formula (IA), (for example, if $R^2$ is methyl, $R^9$ is hydrogen or if $R^2$ is ethyl, $R^9$ is methyl) or, when $R^2$ is methyl, $R^9$ may be alkoxy, for example ethoxy. Suitable reducing agents are known to those skilled in the art and include diborane and lithium aluminium hydride.

(6) A further method, for the preparation of compounds of formula (I) where $R^2$ is hydrogen and $R^1$ is other than hydrogen, comprises hydrolysis of a compound of formula (IX),

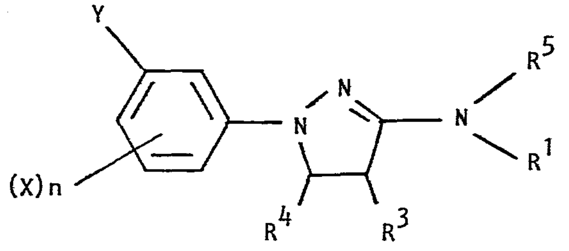

(IX)

wherein Y, X, n, $R^1$, $R^3$ and $R^4$ are as defined in formula (I), $R^5$ is $C_{1-4}$ acyl or alkoxycarbonyl provided that $R^1$ is not hydrogen.

Compounds of formula (IX) may be prepared by reaction of compounds of formula (X), with $R^{10}Z$.

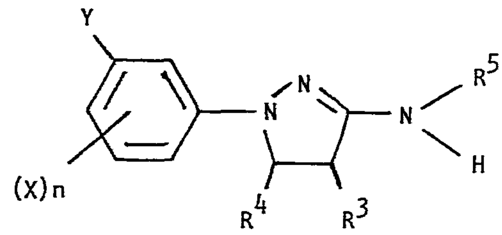

(X)

wherein Y, X, n, $R^3$ and $R^4$, are as defined for formula (I); $R^5$ is $C_{1-4}$ acyl or alkoxycarbonyl $R^{10}$ is alkyl, alkenyl, alkynyl or aralkyl of 7-10 carbon atoms and Z is halogen or arylsulphinyloxy, in the presence of a suitable base eg. sodium hydride, and an inert solvent eg, dimethyl formamide to form a compound of formula (IX) wherein $R^1$ is $R^{10}$ as hereinbefore defined.

Compounds of formula (X) may be prepared by reaction of compounds of formula (I) wherein both $R^1$ and $R^2$ are hydrogen, with an acylating agent, for example, acetic anhydride in acetic acid.

This method is particularly preferred for compounds of formula (I) wherein $R^2$ alkenyl or alkynyl.

(7) A yet further method wherein Y is alkylsulphinyl, arylsulphinyl, alkylsulphonyl, or arylsulphonyl, comprises oxidation of a compound of formula (XI)

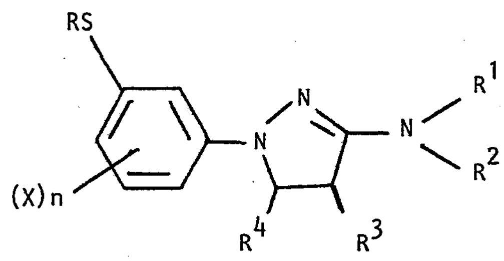

(XI)

wherein, R is alkyl, or aryl, and X, n, $R^1$, $R^2$, $R^3$ and $R^4$ are as defined for compound (I).

The compounds of formula (I) may be used in the relief of rheumatoid arthritis, rheumatoid spondylitis, osteoarthritis, gouty arthritis and other arthritic conditions, inflammed joints, psoriasis, eczema, other inflammatory skin conditions, inflammatory eye conditions including conjunctivitis, pyresis and other conditions associated with inflammation and pain. Such other conditions associated with inflammation include the reduction of tissue necrosis in chronic inflammation, the suppression of tissue rejection following transplant surgery and ulcerative colitis.

The compounds of formula (I) may also be used in the treatment or prophylaxis of allergic conditions and airway inflammatory conditions such as asthma and of asthma having a non-allergic origin and bronchitis. The compounds may also be useful as antispasmogenic agents.

Thus the present invention further provides a compound of formula (I) or a pharmacologically acceptable acid addition salt thereof for use in a method of inhibiting the lipoxygenase and cyclooxygenase pathways of the arachadonic acid metabolism in a mamml such as man. The invention also provides a compound of formula (I) or a pharmacologically acceptable acid addition salt thereof for use in a method of prophylaxis or treatment, by therapy, of a mammal such as man, especially for the therapy of any disease or condition specified herein.

The amount required of a compound of formula (I) (hereinafter referred to as the active ingredient) for therapeutic effect will, of course, vary both with the particular compound, the route of administration and the mammal under treatment and the particular disorder or disease concerned. A suitable dose of a compound of formula (I) for a mammal suffering from an inflammatory, painful or pyretic condition as defined hereinbefore is 0.1 µg-500mg of base per kilogram bodyweight. In the case of systemic administration, the dose may be in the range 0.5 to 500 mg of base per kilogram bodyweight, the most preferred dosage being 0.5 to 50 mg/kg of mammal bodyweight for example 5 to 25 mg/kg; administered two or three times daily. In the case of topical administration, eg. to the skin or eye, a suitable dose may be in the range 0.1ng - 100µg of base per kilogram, typically about 0.1 µg/Kg.

In the case of the treatment or prophylaxis of inflammatory airway conditions, a suitable anti-asthmatic dose of a compound of formula (I) is 1 mg to 10 mg of base per kilogram, the most preferred dosage being 1 mg to 5 mg/kg of mammal bodyweight, for example from 1 to 2 mg/kg.

While it is possible for an active ingredient to be administered alone, it is preferable to present it as a pharmaceutical formulation comprising a compound of formula (I) or a pharmacologically acceptable acid addition salt thereof and a pharmaceutically acceptable carrier therefor. Such formulations consitute a further feature of the present invention. Conveniently, the active ingredient comprises from 0.1% to 99.9% by weight of the formulation. Conveniently, unit doses of a formulation contain between 0.1 mg and 1 g of the active ingredient. For topical administration, the active ingredient preferably comprises from 1% to 2% by weight of the formulation but the active ingredient may comprise as much as 10% w/w. Formulations suitable for nasal or buccal administration, (such as self-propelling powder dispensing formulations described hereinafter), may comprise 0.1 to 20% w/w, for example 2% w/w of active ingredient.

The formulations, both for veterinary and for human medical use, of the present invention comprise an active ingredient in association with a pharmaceutically acceptable carrier therefor and optionally other therapeutic ingredient(s). The carrier(s) must be 'acceptable' in the sense of being compatible with the other ingredients of the formulations and not deleterious to the recipient thereof.

The formulations include those in a form suitable for oral, ophthalmic, rectal, parenteral (including subcutaneous, intramuscular and intravenous), intra-articular, topical, nasal or buccal administration.

The formulations may conveniently be presented in unit dosage form and may be prepared by any of the methods well known in the art of pharmacy. All methods include the step of bringing the active ingredient into association with the carrier which constitutes one or more accessory ingredients. In general, the formulations are prepared by uniformly and intimately bringing the active ingredient into association with a liquid carrier or a finely divided solid carrier or both, and then, if necessary, shaping the product into the desired formulation.

Formulations of the present invention suitable for oral administration may be in the form of discrete units such as capsules, cachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion. The active ingredient may also be in the form of a bolus, electuary or paste.

A tablet may be made by compressing or moulding the active ingredient optionally with one or more accessory ingredients. Compressed tablets may be prepared by compressing, in a suitable machine, the active ingredient in a free-flowing form such as a powder or granules, optionally mixed with a binder, lubricant, inert diluent, surface active or dispersing agent. Moulded tablets may be made by moulding, in a suitable machine, a mixture of the powdered active ingredient and a suitable carrier moistened with an inert liquid diluent.

Formulations for rectal administration may be in the form of a suppository incorporating the active ingredient and a carrier such as cocoa butter, or in the form of an enema.

Formulations suitable for parenteral administration conveniently comprise a sterile aqueous preparation of the active ingredient which is preferably isotonic with the blood of the recipient.

Formulations suitable for intra-articular administration may be in the form of a sterile aqueous preparation of the active ingredient which may be in microcrystalline form, for example, in the form of an aqueous microcrystalline suspension. Liposomal formulations or biodegradable polymer systems may also be used to present the active ingredient for both intra-articular and ophthalmic administration.

Formulations suitable for topical administration include liquid or semi-liquid preparations such as liniments, lotions, applications; oil-in-water or water-in-oil emulsions such as creams, ointments or pastes; or solutions or suspensions such as drops. For example, for ophthalmic administration, the active ingredient may be presented in the form of aqueous eye drops as, for example, a 0.1 - 1.0% solution.

Formulations suitable for administration to the nose or buccal cavity include powder, self-propelling and spray formulations such as aerosols and atomizers. The formulations, when dispersed, preferably have a particle size in the range of 10 to 200μ.

Formulations of the present invention may also be in the form of an aqueous or dilute alcoholic solution, optionally a sterile solution, of the active ingredient for use in a nebuliser or atomiser, wherein an accelerated air steam is used to produce a fine mist consisting of small droplets of the solution. Such formulations usually contain a flavouring agent such as saccharin sodium and a volatile oil. A buffering agent and a surface active agent may also be included in such a formulation which should also contain a preservative such as methylhydroxybenzoate.

Other formulations suitable for nasal administration include a coarse powder having a particle size of 20 to 500 microns which is administered in the manner in which snuff is taken i.e. by rapid inhalation through the nasal passage from a container of the powder held close up to the nose.

In addition to the aforementioned ingredients, the formulations of this invention may include one or more additional ingredients such as diluents, buffers, flavouring agents, binders, surface active agents, thickeners, lubricants, preservatives eg. methylhydroxybenzoate (including anti-oxidants), emulsifying agents and the like.
Any other therapeutic ingredient may comprise one or more of the following: antibiotic, anti-fungal and anti-viral agents.

According to the present invention there are therefore provided:-

(a) a novel compound of formula (I) or an acid addition salt thereof;

(b) a method for preparing a compound of formula (I) or a pharmacologically acceptable acid addition salt thereof;

(c) a pharmaceutical formulation comprising a non-toxic, effective arachidonic acid oxygenation inhibitory amount of a compound of formula (I) or a pharmacologically acceptable acid addition salt thereof and a pharmaceutically acceptable carrier therefor;

(d) a method for preparing such formulations;

(e) a method for the inhibition of the lipoxygenase and cyclooxygenase pathways of arachidonic acid metabolism comprising the administration of a non-toxic, effective, inhibitory amount of a compound of formula (I) or a pharmacologically acceptable acid addition salt thereof; and

(f) a method for the prophylaxis or treatment of inflammation in a mammal, including man, comprising the administration to said mammal of a non-toxic, effective anti-inflammatory amount of a compound of formula (I) or a pharmacologically acceptable acid addition salt thereof;

(g) a method for the prophylaxis or treatment of pain in a mammal, including man, comprising the administration to said mammal of a non-toxic effective analgesic amount of a compound of formula (I) or a pharmacologically acid addition salt thereof;

(h) a method for the prophylaxis or treatment of pyresis in a mammal, including man, comprising the administration to said mammal of a non-toxic, effective anti-pyretic amount of a compound of formula (I) or a pharmacologically acceptable acid addition salt thereof;

(i) a method for the prophylaxis or treatment of asthma in a mammal, including man, comprising administration to said mammal of a non-toxic, effective, anti-asthmatic amount of a compound of formula (I) or a pharmacologically acceptable acid addition salt thereof;

(j) a compound of formula (I) or a pharmacologically acceptable acid addition salt thereof for use in medicine in the inhibition of the lipoxygenase and cyclooxygenase pathways of arachidonic acid metabolism.

The following Examples are provided by way of an illustration of the present invention and should in no way be construed as a limitation thereof. All temperatures indicated are in degrees Celsius.

Example 1 : Preparation of 3-amino-1-(3-methylthiophenyl)-2-pyrazoline

A. Preparation of 3-methylthiophenyl hydrazine

A solution of sodium nitrite (10.54g) in water (53ml) was added dropwise to a suspension of 3-methylthioaniline hydrochloride (24.37g) in concentrated hydrochloric acid (41.3ml) at $0^0$. The mixture was stirred for 1 hr at $0^0$, filtered, and the filtrate was stirred at $0^0$ while a solution of stannous chloride dihydrate (93.78g) in concentrated hydrochloric acid (96ml) was added dropwise. A precipitate formed which was collected, suspended in dichloromethane and cautiously basified with sodium hydroxide solution (10N). Isolation with dichloromethane, afforded 3-methylthiophenyl hydrazine (11.15g) as a pale yellow oil, b.p. $118^0$/0.25 mm.

B. Preparation of 3-amino-1-(3-methylthiophenyl)-2-pyrazoline

3-Methylthiophenyl hydrazine (1g) in SVM (2 ml) was added slowly to a solution of sodium (0.025g) in ethanol (2ml) under nitrogen. The solution was cooled to $0^0$, acrylonitrile (0.3ml) added, and the mixture was heated for 5 hr at $80^0$. The reaction mixture was then allowed to cool to precipitate 3-amino-1-(3-methylthiophenyl)-2-pyrazoline. Treatment of the crude product, with hydrochloric acid followed by recrystallisation from 2-propanol, afforded the hydrochloride salt of 3-amino-1-(3-methylthiophenyl)-2-pyrazoline, m.p. 177-$179^0$.

Example 2 : Preparation of N-[1-(3-methylthiophenyl)-2-pyrazolin-3-yl]formamide

Acetic anhydride (9ml) was added dropwise to a solution of 3-amino-1-(3-methylthiophenyl)- 2-pyrazoline (6g) in 98-100% formic acid (30ml) at $60^0$. The mixture was stirred for 0.5 hr at $60^0$, cooled to room temperature and the crude product was precipitated by the dropwise addition of water. The crude product was isolated, dried and recrystallised from benzene to yield N-[1-(3-methylthiophenyl)-2-pyrazolin-3-yl] formamide, 2.67g, m.p. 125-$127^0$.

Example 3 : Preparation of 3-methylamino-1-(3-methylthiophenyl)-2-pyrazoline hydrobromide

A suspension of N-[1-(3-methylthiophenyl)-2-pyrazolin-3-yl]formamide (0.85g) in ether (20ml) was added to lithium tetrahydridoaluminate (0.43g) in ether (40 ml) under an atmosphere of nitrogen, and the mixture was heated under reflux for 45 mins. The reaction mixture was allowed to cool to ambient temperature, water was cautiously

added, and the product 3-methylamino-1-(3-methylthiophenyl)- 2-pyrazoline was isolated with ether. Treatment of the product with hydrobromic acid, followed by trituration with ethyl acetate afforded 3-methylamino-1-(3-methylthiophenyl)-2-pyrazoline hydrobromide, m.p. 177-179$^{0}$.

Example 4 : Preparation of 3-amino-1-[3-(trifluoromethylsulphonyl)phenyl]-2-pyrazoline

A) Preparation of 3-nitrophenyl thiocyanate

A solution of sodium nitrite (11.7 g) in water 33ml was added dropwise to 3-nitroaniline (23g) in concentrated sulphuric acid (52 g) and water (25 ml) at 0$^{0}$. The intermediate diazonium salt was then treated successively at -10$^{0}$ with solutions of 1M cupric sulphate (41.7 g), ferrous sulphate (70 g) in water (167 ml), and potassium thiocyanate (17.8 g) in water (37 ml). The mixture was then allowed to warm to ambient temperature and stand at ambient temperature for 17 hours. Isolation of neutral material with ether, afforded 3-nitrophenyl thiocyanate (16.8 g), b.p. 107$^{0}$/0.2 mm.

B) Preparation of methyl 3-nitrophenyl sulphide

A solution of potassium hydroxide (16.8 g) in water (16.8 ml) was added cautiously to a solution of 3-nitrophenyl thiocyanate (16.8 g) in methanol (132 ml). The mixture was heated under reflux for 1 hour and then poured into water (800 ml) with vigorous stirring. The resultant suspension was heated to 50$^{0}$, and the pH kept above 8 while dimethyl sulphate (12.6 ml) was added dropwise. The reaction mixture was then allowed to cool and stand at ambient temperature overnight. Isolation of the product with ether, afforded methyl 3-nitrophenyl sulphide, 13.17 g, b.p. 72-74$^{0}$/0.08 mm.

C) Preparation of 3-nitrophenyl trichloromethyl sulphide

Chlorine gas was passed into a solution of methyl 3-nitrophenyl sulphide (13.17 g) in chloroform (22 ml) at 0$^{0}$, while the reaction mixture was irradiated with an incandescent lamp. Passage of chlorine was continued until the mixture crystallised (ca. 20 mins), and the mixture then allowed to stand for 1 hour, during which hydrogen chloride was evolved. The solvent was then removed, the residue was dissolved in chloroform, and chlorine was passed through the solution at 0$^{0}$ for a further hour. Evaporation of the solvent, followed by recrystallisation of the crude product from petroleum ether (60-80$^{0}$) afforded 3-nitrophenyl trichloromethyl sulphide (19.2 g), m.p. 71-72$^{0}$.

D) Preparation of 3-nitrophenyl trifluoromethyl sulphide

A mixture of 3-nitrophenyl trichloromethyl sulphide (19.2 g) and antimony trifluoride (19.2 g), was heated to $100^0$ when a vigorous reaction occurred. The mixture at $100^0$, was then distilled at reduced pressure (water pump) to afford the crude product which was isolated with ether. The organic phase was washed repeatedly with 6N hydrochloric acid, then distilled to provide 3-nitrophenyl trifluoromethyl sulphide, 5.85 g, b.p. $114-115^0/14$ mm.

E) Preparation of 3-nitrophenyl trifluoromethyl sulphone

A mixture of 3-nitrophenyl trifluoromethyl sulphide (5.84 g), chromium trioxide (3.73 g), concentrated sulphuric acid (3.73 g) and water (8.5 ml) was heated overnight in a sealed tube. Steam distillation and isolation with ether followed by recrystallisation from ethanol afforded 3-nitrophenyl trifluoromethylphenyl sulphone, 4.61 g, m.p. $58^0$.

F) Preparation of 3-(trifluoromethylsulphonyl)aniline

A solution of stannous chloride dihydrate (25.85 g) in concentrated hydrochloric acid (19 ml) was added dropwise to 3-nitrophenyl trifluoromethyl sulphone (4.48 g) in SVM at $60-70^0$. The mixture was heated under reflux for 1 hour, and the solvent was removed by distillation. To the residue, was added ice, and 10 N sodium hydroxide solution. The resultant solution was extracted with toluene, the solvent was removed in vacuo, and the resultant residue was recrystallised from light petroleum ($60-80^0$) to yield 3-(trifluoromethylsulphonyl)aniline.

G) Preparation of 3-(trifluoromethylsulphonyl)phenyl hydrazine

By a method analogous to that described in example (IA), 3-(trifluoromethylsulphonyl)aniline, was converted to 3-(trifluoromethylsulphonyl)phenyl hydrazine m.p. $81-82^0$ (recrystallised from toluene).

H) Preparation of 3-amino-1-[3-(trifluoromethylsulphonyl)phenyl]-2-pyrazoline

By a method analogous to that described in example 1B, 3-(trifluoromethylsulphonyl)phenyl hydrazine was converted to 3-amino-1-[3-(trifluoromethylsulphonyl)phenyl]-2-pyrazoline m.p. $140-143^0$, after recrystallisation from ethyl acetate - light petroleum ($60-80^0$).

Example 5 : Preparation of 3-amino-1-(3-methoxyphenyl)-2-pyrazoline

A) Preparation of 3-[(3-methoxyphenyl)amino]propionitrile

A mixture of 3-anisidine (12.3 g), acrylonitrile (5.3 g) and cupric acetate (2 g) was heated for 3 hours at 90$^{o}$ then cooled to ambient temperature. The solid that formed was dissolved in dichloromethane, filtered and passed rapidly over a short column of silica gel. Evaporation of solvent in vacuo from the combined eluates afforded 3-[(3-methoxyphenyl)amino]propionitrile 5.7 g m.p. 78-79$^{o}$.

B) Preparation of 3-amino-1-(3-methoxyphenyl)-2-pyrazoline

A solution of sodium nitrite (2 g) in water (20 ml) was added dropwise to 3-[(3-methoxyphenyl)amino]propionitrile (5 g) in concentrated hydrochloric acid (13 ml) and water (75 ml) at 0$^{o}$. The reaction mixture was warmed to ambient temperature over 1 hour, and the aqueous phase was decanted. The residual oil was dissolved in acetic acid (50 ml), heated to 80$^{o}$, and zinc dust (6 g) was added in portions. The mixture was heated for a further 15 mins, cooled, and the product was isolated with dichloromethane. Recrystallisation of the crude product from light petroleum afforded 3-amino-1-(3-methoxyphenyl)-2-pyrazoline, 0.3 g, m.p. 128-129$^{o}$.

Example 6: Preparation of N-[1-(3-methylthiophenyl)-2-pyrazolin-3-yl] acetamide

A mixture of 3-amino-1-(3-methylthiophenyl)-2-pyrazoline (1 g) and acetic anhydride (5 ml) was stirred for 1 hr at room temperature to precipitate N-[1-(3-methylthiophenyl)-2-pyrazolin-3-yl]acetamide (0.4g), m.pt. 183-185$^{o}$ after recrystallisation from ethanol.

Example 7: Preparation of 1-(3-Methylthiophenyl)-3-(2-propynylamino)-2-pyrazoline hydrochloride

A. Preparation of N-[1-(3-methylthiophenyl)-2-pyrazolin-3-yl]-N-(2-propynyl)formamide

A 50% dispersion of sodium hydride in mineral oil (0.39g) was added to N-[1-(3-methylthiophenyl)-2-pyrazolin-3-yl]formamide (1.9g) in N,N-dimethylformamide (30ml), and the mixture was stirred until the evolution of hydrogen ceased. Propargyl bromide (80% in toluene; 1.1 ml) was added, and mixture was stirred for 1 hr at the ambient temperature (to pH ca. 7), then quenched with ice-water to furnish N-[1-(3-

methylthiophenyl)-2-pyrazolin-3-yl]-N-(2-propynylamino)formamide (1.8g), m.pt. 119-122$^{0}$ after a wash with light petroleum (b.pt. 60-80$^{0}$).

B. Preparation of 1-(3-Methylthiophenyl)-3-(2-propynylamino)-2-pyrazoline Hydrochloride

A solution of N-[1-(3-methylthiophenyl)-2-pyrazolin-3-yl]-N-(2-propynyl)-formamide (1.7g) in 1.0N-sodium hydroxide (6.8ml) and ethanol (ca. 90 ml) was stirred for 2 hr at room temperature. Evaporation and isolation with ether afforded 1-(3-methylthiophenyl)-3-(2-propynylamino)-2-pyrazoline as an oil. The hydrochloride salt (0.7g) decomposed at above 135$^{0}$.

Example 8: Preparation of 1-(3-methylthiophenyl)-3-(2-propenylamino)-2-pyrazoline hydrochloride

A. Preparation of N-[1-(3-methylthiophenyl)-2-pyrazolin-3-yl]-N-(2-propenyl) acetamide

By following the procedure of Example 7 but using allyl bromide in place of propargyl bromide, and N-[1-(3-methylthiophenyl)-2-pyrazolin-3-yl]acetamide in place of the corresponding formamido-compound, there was obtained N-[1-(3-methylthiophenyl)-2-pyrazolin-3-yl]-N-(2-propenyl)acetamide, m.pt. 51-52.5$^{0}$ (from methanol-water).

B. Preparation of 1-(3-methylthiophenyl)-3-(2-propenylamino)-2-pyrazoline hydrochloride

By following the procedure of Example 8 but using N-[1-(3-methylthiophenyl)-2-pyrazolin-3-yl]-N-(2-propenyl)acetamide, there was obtained 1-(3-methylthiophenyl)-3-propenylamino)-2-pyrazoline as an oil. The hydrochloride salt had m.pt 132-134$^{0}$ (from methanol-ether).

Example 9: Preparation of 3-(4-methoxybenzylamino)-1-(3-methylthiophenyl)-2-pyrazoline

A. Preparation of N-[1-(3-methylthiophenyl)-2-pyrazolin-3-yl]-N-(4-methoxybenzyl) acetamide.

By following the procedure of Example 8A but using 4-methoxybenzyl chloride in place of the allyl bromide, there was obtained N-[1-(3-methylthiophenyl)-2-pyrazolin-3-yl]-N-(4-methoxybenzyl)acetamide as an oil which was purified by chromatography over silica gel (elution with chloroform).

B. Preparation of 3-(4-Methoxybenzylamino)-1-(3-methylthiophenyl)-2-pyrazoline

By following the procedure of Example 8B but using N-[1-(3-methylthiophenyl)-2-pyrazolin-3-yl]-N-(4-methoxybenzyl)acetamide as the starting material, there was obtained 3-(4-methoxybenzylamino)-1-(3-methylthiophenyl)-2-pyrazoline m.pt. 82-84$^{0}$ (from ethanol-water).

Pharmaceutical Formulations

In the following examples the 'Active Ingredient' may be any compound of formula (I) or a pharmacologically acceptable acid addition, for example 3-amino-1-(3-methylthiophenyl)-2-pyrazoline.

EXAMPLE A : Tablet

| | In one tablet |
|---|---|
| Active Ingredient | 5.0 mg |
| Lactose | 82.0 mg |
| Starch | 10.0 mg |
| Povidone | 2.0 mg |
| Magnesium Stearate | 1.0 mg |

Mix together the active ingredient, lactose and starch. Granulate the powders using a solution of povidone in purified water. Dry the granules, add the magnesium stearate and compress to produce tablets, 100 mg per tablet.

EXAMPLE B : Ointment

| | |
|---|---|
| Active Ingredient | 1.0 g |
| White Soft Paraffin | to 100.0 g |

Disperse the active ingredient in a small volume of the vehicle. Gradually incorporate this into the bulk to produce a smooth, homogeneous product. Fill into collapsible metal tubes.

EXAMPLE C : Cream for Topical Use

| | |
|---|---|
| Active Ingredient | 1.0 g |
| Polawax GP 200 | 20.0 g |
| Lanolin Anhydrous | 2.0 g |
| White Beeswax | 2.5 g |
| Methyl Hydroxybenzoate | 0.1 g |
| Distilled Water | to 100.0 g |

Heat the Polawax, beeswax and lanolin together at 60°C. Add a solution of methyl hydroxybenzoate. Homogenise using high speed stirring. Allow the temperature to fall to 50°. Add and disperse the active ingredient. Allow to cool with slow speed stirring.

EXAMPLE D : Lotion for Topical Use

| | |
|---|---|
| Active Ingredient | 1.0 g |
| Sorbitan Monolaurate | 0.6 g |
| Polysorbate 20 | 0.6 g |
| Cetostearyl Alcohol | 1.2 g |
| Glycerin | 6.0 g |
| Methyl Hydroxybenzoate | 0.2 g |
| Purified Water B.P. | to 100.00 ml |

The methyl hydroxybenzoate and glycerin were dissolved in 70 ml of the water at 75°C. The sorbitan monolaurate, Polysorbate 20 and cetostearyl alcohol were melted together at 75°C and added to the aqueous solution. The resulting emulsion was homogenised, allowed to cool with continuous stirring and the active ingredient added as a suspension in the remaining water. The whole was stirred until homogeneous.

EXAMPLE E : Eye Drops

| | |
|---|---|
| Active Ingredient | 0.5 g |
| Methyl Hydroxybenzoate | 0.01 g |
| Propyl Hydroxybenzoate | 0.04 g |
| Purified Water B.P. | to 100.00 ml |

The methyl and propyl hydroxybenzoates were dissolved in 70 ml purified water at 75° and the resulting solution then allowed to cool. The active ingredient was added next and the solution made up to 100 ml with purified water. The solution was sterilised by filtration through a membrane filter 0.22 $\mu$ pore size and packed aseptically into suitable sterile containers.

EXAMPLE F : Injection Solution

| | |
|---|---|
| Active Ingredient | 10.0 mg |
| Water for Injections B.P. | to 1.0 ml |

The active ingredient was dissolved in half of the Water for Injection and then made up to volume and sterilised by filtration. The resulting solution was distributed into ampoules under a aseptic conditions.

EXAMPLE I : Inhibition of Lipoxygenase and Cyclooxygenase

In an enzyme assay according to a method closely based on that of P. Borgeat et al (J. Biol. Chem., 251 (1976) 7816-20) compounds of the invention were found to have an $IC_{50}$ (uM) for inhibition of each of lipoxygenase and cyclo-oxygenase as indicated in Table I.

TABLE I

| Compound | $IC_{50}$ (uM) | |
|---|---|---|
| Example No. | Cyclooxygenase | Lipoxygenase |
| 1 | 4.7 | 5.5 |
| 3 | 10 | 0.5 |
| 7 | >1 | 0.1 |

Example II : In vivo Activity

The compound of Example 1 was subjected to the following tests. Results are given in Table II as $ED_{50}$ mg/kg upon oral administration. Results are also provided by way of comparison for the compound 3-methylamino-1-(3-trifluoromethylphenyl)-2-pyrazoline (Compound A) which is disclosed in European Specification 55 418.

1) Prostaglandin synthesis and leucocyte accumulation

Inflammatory exudates were collected 24 hours after the subcutaneous implantation in male rats (180-220g) of sterile polyester sponges impregnated with 2% carrageenin. Prostaglandin $E_2$ concentrations in sponge exudates were measured by bioassay and total leucocyte numbers were estimated using counting chambers and phase contrast microscopy. Drugs were administered orally in aqueous solution to groups of five animals at the time of sponge implantation, 5-8 hours later and 3 hours before sponge removal. Results are expressed as a percentage of control values obtained from similar groups of animals receiving vehicle alone. $ED_{50}$ values were calculated for active compounds. The full dose-response data for lead compounds were subjected to regression analysis and $ED_{50}$ values with 95% confidence limits have been obtained.

2) Oedema

Oedema was induced by the sub-plantar injection of 2% carrageenin (0.1 ml) in the hind paws of female rats (100-130g). Swelling was measured 1-4 hours following carrageenin injection using a micrometer gauge. Drugs in aqueous solution were administered orally to groups of five rats at the time of carrageenin injection. Mean swelling was expressed as a percentage of control values obtained from similar groups of animals receiving vehicle alone.

3) Pain

Writhing was induced in groups of 5 female mice (20-25g) following the intraperitoneal injection of phenylbenzoquinone (PBQ; 2.5 mg/kg). Anti-nociceptive effects of drugs were assessed by observing the reduction in writhing responses 10-12½ min after PBQ injection. Drugs were administered orally in aqueous solution 1 hour before PBQ injection and drug effects were expressed as a percentage of control values obtained from animals receiving vehicle alone.

Table II

$ED_{50}$ (mg/kg p.o.)

| Test | Compound of Example 1 | Compound A |
|---|---|---|
| Prostaglandin Production (rat) | 14 | 20 |
| Leucocyte Migration (rat) | 6.5 | 20 |
| Carrageenin Oedema (rat) | 20 | 26 |
| PBQ Writhing (mouse) | 15 | 20 |

Example III: Toxicity

The compound of Example 1 was found not to be ulcerogenic in the rat at doses in the range 50-100 mg/kg.

RMW/KMS/DC14/16.01.84

Claims:-

1) A compound of formula (I)

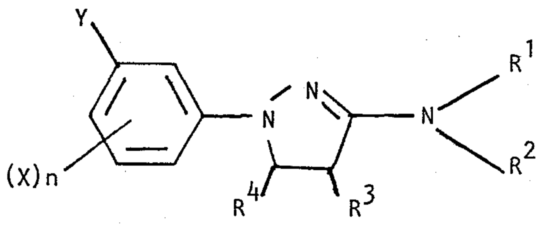

(I)

wherein

Y is a group selected from alkylthio, alkenylthio, arylthio, alkylsulphinyl, alkenylsulphinyl, arylsulphinyl, alkylsulphonyl, alkenylsulphonyl, arylsulphonyl, alkoxy, alkenyloxy, or aryloxy, in which group any hydrogen atom is optionally replaced by a halogen atom;

each X is the same or different and is independently selected from alkylthio, alkenylthio, arylthio, alkylsulphinyl, arylsulphinyl, alkenylsulphinyl, alkylsulphonyl, alkenylsulphonyl, arylsulphonyl, alkoxy, alkenyloxy, aryloxy, alkyl, alkenyl, aryl, trihaloalkyl, or halogen;

n is 0 to 4;

$R^1$ is hydrogen, alkyl, alkenyl, alkynyl, aralkyl or aryl;

$R^2$ is hydrogen, alkyl, acyl of 1-4 carbon atoms, aroyl of 6-10 carbon atoms or alkoxycarbonyl; or

$R^1$ and $R^2$ together represent =CH- 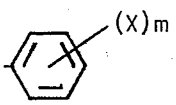 where X is a suitable substituent and m is 0-5; and

$R^3$ and $R^4$ are the same or different and each represents hydrogen or alkyl

provided that when $R^1$ is other than hydrogen, $R^2$ is not acyl or aroyl;

and pharmacologically acceptable acid addition salts thereof.

2) A compound of formula (I) as defined in claim 1, wherein $R^1$ is other than aralkyl and $R^2$ is other than aroyl and pharmacologically acceptable acid addition salts thereof.

3) A compound as claimed in claim 1 or claim 2, wherein both $R^1$ and $R^2$ are hydrogen, and pharmacologically acceptable acid addition salts thereof.

4) A compound as claimed in any preceding claim, where in Y is alkylthio and/or n is 0, and $R^3$ and $R^4$ are hydrogen, and pharmacologically acceptable acid addition salts thereof.

5) A compound as claimed in any of claims 1-3, wherein Y is alkylthio or trihalo-alkysulphonyl, n is 0, $R^1$ is other than aryl, $R^2$ is other than alkoxycarbonyl and $R^3$ and $R^4$ are both hydrogen, and pharmacologically acceptable acid addition salts thereof.

6) A compound as claimed in any of claim 1 and claims 3-5 when not dependent on claim 2, wherein Y is alkylthio, n is 0, $R^1$ is hydrogen, alkyl, alkenyl, alkynyl, or benzyl (wherein benzyl is optionally substituted in the benzene ring by one or more suitable substituents $R^2$ is other than alkoxycarbonyl and $R^3$ and $R^4$ are both hydrogen, and pharmacologically acceptable acid addition salts thereof.

7) 1-(3-methylthiophenyl)-3-amino-2-pyrazoline, and its pharmacologically acceptable acid addtion salts.

8) A compound of formula (I) as defined in claim 1, selected from the following and their pharmacologically acceptable acid addition salts:-

N-[1-(3-methylthiophenyl)-2-pyrazolin-3-yl]formamide
3-methylamino-1-(3-methylthiophenyl)-2-pyrazoline
3-amino-1-[3-(trifluoromethylsulphonyl)phenyl]-2-pyrazoline
3-amino-1-(3-methoxyphenyl)-2-pyrazoline
N-[1-(3-methylthiophenyl)-2-pyrazolin-3-y] acetamide
1-(3-methylthiophenyl)-3-(2-propynylamino)-2-pyrazoline
1-(3-methylthiophenyl)-3-(2-propenylamino)-2-pyrazoline
3-(4-methoxybenzylamino)-1-(3-methylthiophenyl)-2-pyrazoline

9) A pharmaceutical formulation comprising a compound as claimed in any preceding claim or a pharmacologically acceptable acid addition salt thereof, and a pharmaceutically acceptable carrier therefor.

10) A compound of formula (I) as claimed in claim 1 or a pharmacologically acceptable acid addition salt thereof for use in a method of inhibiting the lipoxygenase and cyclooxygenase pathways of the arachadonic acid metabolism in a mamml such as man.

11) A compound of formula (I) as claimed in claim 1, or a pharmacologically acceptable acid addition salt thereof for use in a method of prophylaxis or treatment, by therapy, of a mammal such as man.

12) A compound of formula (I) as claimed in claim 1, or a pharmacologically acceptable acid addition salt thereof for use in relief of rheumatoid arthritis, rheumatoid spondylitis, osteoarthritis, gouty arthritis and other arthritic conditions, inflammed joints, psoriasis, eczema, other inflammatory skin conditions, inflammatory eye conditions including conjunctivitis, pyresis and other conditions associated with inflammation and pain, or for the reduction of tissue necrosis in chronic inflammation, the suppression of tissue rejection following transplant surgery or the prophylaxis or treatment of ulcerative colitis.

13) A compound of formula (I) as claimed in claim 1, or a pharmacologically acceptable acid addition salt thereof, for use in the treatment or prophylaxis of allergic conditions and airway inflammatory conditions such as asthma and of asthma having a non-allergic origin and bronchitis.

14) A compound of formula (I) as claimed in claim 1, or a pharmacologically acceptable acid addition salt thereof, for use as an antispasmogenic agent.

European Patent Office

# EUROPEAN SEARCH REPORT

Application number

EP 84 10 1394

| Category | Citation of document with indication, where appropriate, of relevant passages | Relevant to claim | CLASSIFICATION OF THE APPLICATION (Int. Cl. 3) |
|---|---|---|---|
| X | EP-A-0 056 466 (WELLCOME) * Claims * | 1-14 | C 07 D 231/06<br>A 61 K 31/415 |
| D,X | EP-A-0 055 418 (WELLCOME) * Claims * | 1-14 | |
| X | EP-A-0 022 578 (WELLCOME) * Claims * | 1-14 | |
| Y | GB-A- 743 505 (ILFORD) * Page 1, formula II and page 1, lines 46-49 * | 1-9 | |
| A | DE-A-2 038 919 (CHINOIN GYOGYSZER) * Claims * | 1-9 | |
| | | | **TECHNICAL FIELDS SEARCHED (Int. Cl. 3)** |
| | | | C 07 D 231/00 |

The present search report has been drawn up for all claims

| Place of search | Date of completion of the search | Examiner |
|---|---|---|
| THE HAGUE | 15-05-1984 | MAISONNEUVE J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82